# EUROPÄISCHE PATENTSCHRIFT

(11) **EP 1 643 997 B1**
(45) Veröffentlichungstag und Bekanntmachung des Hinweises auf die Patenterteilung: **10.11.2010**
(21) Anmeldenummer: 04740166.6
(22) Anmeldetag: 22.06.2004
(51) Int. Cl.: A61P 3/10, A61K 31/44, A61P 27/06

(54) **FLUPIRTINE-PRÄPARAT ZUR BEHANDLUNG VON NEURODEGENERATIVEN ERKRANKUNGEN DES SEHAPPARATES UND VON DIABETES MELLITUS**
FLUPIRTINE PREPARATION FOR TREATING NEURODEGENERATIVE DISORDERS OF THE VISUAL SYSTEM AND DIABETES MELLITUS
PREPARATION DE FLUPIRTINE-POUR LE TRAITEMENT DE MALADIES NEURODEGENERATIVES DU SYSTEME VISUEL ET DU DIABETE SUCRE

(30) Priorität: 23.06.2003 DE 10328260
(43) Veröffentlichungstag der Anmeldung: 12.04.2006
(73) Patentinhaber: Schmidt, Karl-Georg, 01307 Dresden (DE)
(72) Erfinder: Schmidt, Karl-Georg, 01307 Dresden (DE)
(74) Vertreter: Boeters, Hans Dietrich
(86) Internationale Anmeldenummer: PCT/EP2004/006738
(87) Internationale Veröffentlichungsnummer: WO 2005/000306

(56) Entgegenhaltungen:
- EP-A- 1 199 067
- WO-A-95/05175
- WO-A-97/49398
- US-A- 5 162 311
- OSBORNE N N ET AL: "NEUROPROTECTION IN RELATION TO RETINAL ISCHEMIA AND RELEVANCE TO GLAUCOMA" SURVEY OF OPHTHALMOLOGY, SURVEY OF OPHTHALMOLOGY INC, XX, Bd. 43, Nr. 6, Juni 1999 (1999-06), Seiten S102-S128, XP000986473 ISSN: 0039-6257
- OSBORNE N N ET AL: "THE POTENTIAL OF NEUROPROTECTION IN GLAUCOMA TREATMENT" CURRENT OPINION IN OPHTHALMOLOGY, PHILADELPHIA, PA, US, Bd. 10, Nr. 2, April 1999 (1999-04), Seiten 82-92, XP000870145 ISSN: 1040-8738
- OSBORNE N N ET AL: "IMMUNOHISTOCHEMICAL EVIDENCE FOR FLUPIRTINE ACTING AS AN ANTAGONIST ON THE N-METHYL-D-ASPARTATE AND HOMOCYSTEIC ACID-INDUCED RELEASE OF GABA IN THE RABBIT RETINA" BRAIN RESEARCH, AMSTERDAM, NL, Bd. 667, Nr. 2, 1994, Seiten 291-294, XP000671785 ISSN: 0006-8993
- DISTELHORST J.S.: 'Open-Angle Glaucoma' AMERICAN FAMILY PHYSICIAN Bd. 67, Nr. 9, 01 Mai 2003, Seiten 1937 - 1944
- DREYER E.B.: 'Elevated Glutamate Levels...' Bd. 114, 1996, Seiten 299 - 305

## Beschreibung

Präparate mit einem Gehalt an Flupirtine gehören zum Stand der Technik. Derartige Präparate werden beispielsweise zur Behandlung von Tinnitus (WO02/15907) der Batten-Krankheit (WO01/39760), von Fibromyalgie (WO00/59487), gegen Zellzerstörung durch Apoptose und Nekrose (WO97/49398), einer Beeinträchtigung des haemopoietischen Zellsystems (WO97/17072), als Analgetikum (WO97/14415), gegen neurodegenerative Erkrankungen (WO95/05175), gegen die Creutzfeldt-Jacob-Krankheit (Molecule of the Month, May 2001) oder als Antiphlogistikum (DE 1795858) verwendet. DE 3133519 spricht allgemein von Flupirtine-Arzneimitteln.

Die DE 196 25 582 A1 offenbart die Verwendung von Flupirtine oder dessen Salze zur Herstellung eines Arzneimittels zur Prophylaxe und Therapie von Erkrankungen, die mit unphysiologisch hoher Zellsterberate einhergehen. Hierunter werden einerseits Erkrankungen verstanden, die mit lokalem Gewebstod als schwerste Folge einer örtlichen Stoffwechselstörung beispielsweise in Folge mechanischer, thermischer, toxischer oder Strahlungsbelastung verbunden sind. Im Organismus kann das Absterben von Zellen auf unterschiedliche Wege erfolgen. Während die Apoptose (programmierter Zelltod) einen aktiven Prozess der Zellzerstörung darstellt, ist die Nekrose Folge einer unspezifischen Schädigung insbesondere der Zellmembran. Die DE 196 25 582 A1 führt daneben aus, daß der programmierte Zelltod durch Bcl-2, ein 25 kDa membranständiges Protein mit zirka 240 Aminosäuren gehemmt werden kann. In den Beispielen wird der Einfluß von Flupirtine auf die Expression von Bcl-2 untersucht, und es wurde beobachtet, daß Zellen, die mit Flupirtine behandelt wurden, ein deutlich intensiveres Färbemuster mit Antikörpern gegenüber Bcl-2 zeigen. Die Erfinder der DE 196 25 582 A1 schließen daraus, daß Flupirtine die Bcl-2 Expression induziert.

Die DE 196 25 582 A1 offenbart nicht, daß Apoptose eine Rolle im Sehapparat zukommen könnte.

Die DE 43 27 516 A1 beschreibt die Anwendung von Flupirtine bei neurodegenerativen Erkrankungen, zerebraler Ischämie und epileptischen Anfällen.

Die Autoren Nash und Kollegen führten Studien durch, um zu untersuchen, ob Flupirtine Schäden an den Retina-Ganglienzellen nach Ischämie in einer Ratte verhindern kann. Das von Nash et al. verwendete Modell führt über eine massive Druckerhöhung im Auge (120 mm Hg für eine Stunde) zum vollständigen Unterbinden der Blutzufuhr, was zu einem absoluten Sauerstoff- und Nährstoffmangel führt. Dieser Zustand wird als Ischämie bezeichnet. Eine solche massive Druckerhöhung besitzt mit Ausnahme des so genannten Glaukomanfalls bei Engwinkelglaukomen keine Entsprechung im Menschen. Bei Engwinkelglaukomen kommt es zur Verlegung der Abflußwege des Kammerwassers, wo typische Druckwerte im Bereich von 50-80 mm Hg liegen. Bleibt eine solche seltene Ischämie des Menschen unbehandelt, kommt es innerhalb von 24-48 Stunden zur Erblindung. Die von Nash et al. offenbarten Befunde, daß Flupritine den Tod der Ganglienzellen in der Rattenretina abschwächen könnte, lassen sich nicht auf den Menschen übertragen, weil die Versuchsbedingungen in einem extrem experimentellen Umfeld gewählt wurden. Nash et al. spekulieren deshalb auch in keinster Weise über eine Anwendung beim Menschen. (Nash et al., Brain Research 856 (2000), 236-239).

US 5,162,311 weist darauf hin, dass NMDA Antagonisten durch Inhibition der GH und LH Sekretion nützlich in der Behandlung von Diabetes sein könnten.

WO 95/05175 zeigt NMDA Inhibition durch Flupirtin und schliesst auf therapeutische Wirksamkeit bei degenerativen und ischämischen Erkrankungen der Retina. Retinitis pigmentosa ist nicht erwähnt.

Osborne N N et al., Survey of Ophthalmology 43 Supplement 1, 1999 offenbart einen neuroprotektiven Effekt von NMDA-Antagonisten wie etwa Flupirtin bei Ischämie, beispielsweise im Zusammenhang mit Glaukom und Ischämie der Retina, und schlägt Flupirtine als möglichen Wirkstoff zur Behandlung dieser ischämischen Zustände vor. Die Schlussfolgerungen in D1 beruhen auf den Beobachtungen, dass bei Glaukom Glutamat von der Retina freigesetzt wird, und dass Flupirtine Ischämie-induzierte Schädigungen der Retina in Kaninchen verringert.

Überraschenderweise wurde festgestellt, daß sich ein Präparat mit einem Gehalt an Flupirtine auch bei neurodegenerativen Erkrankungen des Sehapparats, wie Glaukom (Zelltodmechanismus bei Glaukom) oder Diabetes mellitus und Maculopathie, insbesondere altersbedingte Maculopathie, aber auch genetisch bedingte Maculopathien, Retinitis pigmentosa, sowie Apoptose des Sehapparats verwenden läßt.

So betrifft eine Ausführungsform der Erfindung ein pharmazeutisches Präparat enthaltend Flupirtine zur Verwendung in der Therapie und/oder Prophylaxe von Diabetes mellitus, diabetischer Retinopathie, diabetischer Maculopathie, Retinitis pigmentosa und/oder menschlichen Glaukomen, wobei das menschliche Glaukom ein Offenwinkelglaukom ist.

Das erfindungsgemäße pharmazeutische Präparat kann mit einem Gehalt an mindestens einem üblichen Träger und/oder mindestens einem üblichen Hilfsstoff vorgesehen sein.

Das erfindungsgemäße pharmazeutische Präparat kann durch eine orale Applikationsform gekennzeichnet sein.

Ferner kann das erfindungsgemäße Präparat dadurch gekennzeichnet sein, daß es als Tablette oder in einer Kapsel vorgesehen ist.

Ferner kann das erfindungsgemäße Präparat dadurch gekennzeichnet sein, daß es als Retard-Applikationsform vorgesehen ist.

Ferner kann das erfindungsgemäße Präparat dadurch gekennzeichnet sein, daß es als Tablette oder in einer Kapsel mit magensaftresistentem Überzug vorgesehen ist.

Ferner kann das erfindungsgemäße Präparat dadurch gekennzeichnet sein, daß es als Trinklösung vorgesehen ist.

Ferner kann das erfindungsgemäße Präparat dadurch gekennzeichnet sein, daß es als Brausetablette vorliegt.

Ferner kann das erfindungsgemäße Präparat dadurch gekennzeichnet sein, daß es als transdermales therapeutisches System vorgesehen ist, insbesondere als Pflaster.

Ferner kann das erfindungsgemäße Präparat dadurch gekennzeichnet sein, daß es als Tropflösung vorgesehen ist, insbesondere als Augentropfen.

Ferner kann das erfindungsgemäße Präparat dadurch gekennzeichnet sein, daß es als Salbe vorgesehen ist, insbesondere als Augensalbe.

Ferner kann das erfindungsgemäße Präparat dadurch gekennzeichnet sein, daß es als Infusionslösung vorgesehen ist.

Ferner kann das erfindungsgemäße Präparat dadurch gekennzeichnet sein, daß es als Suppositorium vorgesehen ist.

Ferner kann das erfindungsgemäße Präparat dadurch gekennzeichnet sein, daß es als Gel vorgesehen ist.

Eine weitere Ausführungsform der Erfindung betrifft die Verwendung von Flupirtine zur Herstellung eines pharmazeutischen Präparats zur Therapie und/oder Prophylaxe von Diabetes mellitus, diabetischer Retinopathie, diabetischer Maculopathie, Retinitis pigmentosa und/oder menschlichen Glaukomen, wobei das menschliche Glaukom ein Offenwinkelglaukom ist.

Die Erfindung zielt ferner darauf ab, neue Verwendungen von Flupirtine sowie Kombinationspräparate aus Flupirtine und mindestens einem weiteren Wirkstoff zur Therapie und/oder Prophylaxe vorzuschlagen.

Erfindungsgemäß wird diese Aufgabe durch die im folgenden ausgeführten spezifischen Ausführungsformen der Erfindung gelöst.

So kann Flupirtine oder eine Kombination aus Flupirtine und mindestens einem weiterem Wirkstoff zur Therapie und/oder Prophylaxe von Diabetes mellitus, diabetischer Retinopathie, diabetischer Maculopathie, Retinitis pigmentosa und/oder menschlichen Glaukomen verwendet werden. Das menschliche Glaukom ist ein Offenwinkelglaukom.

Bisher ist kein allgemein akzeptiertes Tiermodell für Offenwinkelglaukome entwickelt worden. Das in Nash et al. offenbarte, so genannte "Ischämie Modell", in dem jegliche Blutzufuhr zu den Strukturen des Auges unterbunden wird, ist für vergleichende Untersuchungen ungeeignet und deshalb nicht auf den Menschen übertragbar. In diesem Modell werden 120 mm Hg für eine Stunde angelegt, d.h. 120 mm Hg über dem arteriellen Mitteldruck, was ein extrem unphysiologischer Druck ist. Er führt zur sofortigen Ischämie und so in kürzester Zeit über einen absoluten Sauerstoff- und Nährstoffmangel zum Untergang vieler Zellen im Augeninneren. Hiervon sind beispielsweise das Hornhautendothel, das Trabekelwerk, die Iris, der Ziliarkörper, die Netzhaut, das retinale Pigmentepithel als auch die Aderhaut betroffen.

Im Gegensatz dazu liegt der Augeninnendruck bei Offenwinkelglaukomen vor der Therapie in der Regel bei weniger als 30 mm Hg. Bei Normaldruckglaukomen, die ebenfalls durch die erfindungsgemäße Verwendung von Flupirtine verhindert oder therapiert werden können, liegt er sogar nur bei unter 21 mm Hg, was sich im Bereich der Normalwerte von 10-21 mm Hg bewegt. Glaukome, insbesondere die Offenwinkelglaukome zählen nicht zu Netzhauterkrankungen (Retinaerkrankungen) sondern sind Erkrankungen des Sehnervs bzw. Sehnervkopfes, so daß sich die Behandlung und/oder Prophylaxe auf den Erhalt von Sehnerven richtet, die dann sekundär die Netzhautzellen erhält.

Es hat sich als besonders vorteilhaft herausgestellt, Offenwinkelglaukome mit Flupirtine zu therapieren. Ein besonders überraschender Effekt konnte bei der Behandlung dieser Glaukome festgestellt werden, wenn Flupirtine mit weiteren Arzneimitteln in einem Kombinationspräparat verabreicht wurde. Als besonders vorteilhaft haben sich hierbei blutdrucksenkende Arzneimittel, durchblutungsfördernde Arzneimittel, Vitaminpräparate, Spurenelementen, Mineralstoffen und Radikalfängern gezeigt. Bei den Offenwinkelglaukomen (Augeninnendruck vor Therapie in der Regel unter 30 mm Hg, bei Normaldruckglaukomen unter 21 mm Hg, behandelt mindestens 30 % unter Ausgangswert) ist der Abfluß des Kammerwassers, im Gegensatz zu Engwinkelglaukomen, nicht verlegt, so daß es zu keiner Reduktion oder gar vollständiger Verhinderung der Blutzufuhr kommt. Deshalb verlaufen Offenwinkelglaukome über Jahre.

Bei Diabetes mellitus, der diabetischen Retinopathie sowie bei Retinitis pigmentosa besteht in der Regel ein normaler Augeninnendruck (unter 21 mm Hg), so daß diese Erkrankungen nicht durch massiv erhöhten Augeninnendruck entstehen. Vielmehr liegt Diabetes mellitus und der diabetischen Retinopathie eine Störung des Zucker- bzw. Insulinstoffwechsels zugrunde.

Bei der Retinitis pigmentosa ist eine meist genetisch bedingte Zerstörung des retinalen Pigmentepithel ursächlich. Auch diese Krankheiten verlaufen über Jahre.

Ferner weist das pharmazeutische Präparat in der erfindungsgemäßen Verwendung mindestens einen üblichen Träger und/oder mindestens einen üblichen Hilfsstoff auf.

Das pharmazeutische Präparat kann insbesondere oral appliziert werden. Auf diese Weise können für den Patienten unangenehme Applikationsformen, wie Injektion oder direkte Behandlung des Auges wirkungsvoll vermieden werden.

In einer bevorzugten Ausführungsform liegt das pharmazeutische Präparat als Tablette oder in einer Kapsel vor.

Es ist außerdem bevorzugt, wenn das pharmazeutische Präparat als Retard-Applikationsform vorliegt. Retard-Applikationsform weisen den besonderen Vorteil auf, daß die Abgabe des Wirkstoffs im Organismus des Patienten über einen langen Zeitraum verteilt kontinuierlich erfolgt. So kann eine anfängliche "Überdosierung", die jedoch danach stark abfällt, wirkungsvoll vermieden werden.

Ferner kann die Tablette oder die Kapsel einen magensaftresistenten Überzug aufweisen.

Das pharmazeutische Präparat kann auch als Trinklösung vorliegen.

Ferner kann das pharmazeutische Präparat als Brausetablette vorliegen.

Daneben ist es bevorzugt, wenn das pharmazeutische Präparat als transdermales therapeutisches System, insbesondere als Pflaster, als Tropflösung, insbesondere als Augentropfen, als Salbe, insbesondere als Augensalbe, als Infusionslösung, als Suppositorien, als Gel und/oder als Medikamententräger, insbesondere als ocusert vorliegt.

Medikamententräger (ocuserts), die direkt auf die Bindehaut gelegt werden und konstant Flupirtine sowie gegebenenfalls einen weiteren Wirkstoff konstant abgeben, haben sich als praktisch wertvoll erwiesen.

Ein weiterer Gegenstand der Erfindung ist ein Kombinationspräparat enthaltend Flupirtine und wenigstens einen weiteren Wirkstoff zur Therapie und/oder Prophylaxe von Diabetes mellitus, diabetischer Retinopathie, diabetischer Maculopathie, Retinitis pigmentosa und/oder menschlichen Glaukomen, wobei das menschliche Glaukom ein Offenwinkelglaukom ist.
Schließlich können Flupirtine und wenigstens ein weiterer Wirkstoff zur Herstellung eines Kombinationspräparats verwendet werden, das zur Therapie und/oder Prophylaxe von Diabetes mellitus, diabetischer Retinopathie, diabetischer Maculopathie, Retinitis pigmentosa und/oder menschlichen Glaukomen eingesetzt wird, wobei das menschliche Glaukom ein Offenwinkelglaukom ist.

Nachstehend wird die Erfindung durch Beispiele näher erläutert.

### Beispiel 1 (Glaukom)

Es wurden die Katadolon Kapseln verwendet.

| | |
|---|---|
| Bestandteile: | Flupirtinmaleat |
| | Calciumhydrogenphosphat |
| | Copovidon |
| | Magnesiumstearat |
| | hochdisp. Siliciumdioxid |
| | Gelatine |
| | Farbstoffe: E171, E172 |
| | Natriumdodecylsulfat |

Die Behandlung wurde an einem Patienten durchgeführt der seit über 13 Jahren an einem fortschreitenden Glaukom erkrankt ist, konservativ behandelt wird und mit einer filtrierenden Operation versorgt wurde.

Der Patient wurde 6 Jahre lang mit dem genannten Präparat bei Fortführung der konservativen Therapie behandelt. In diesem Zeitraum kam es zu keiner Befundverschlechterung. Die Behandlung schützte somit vor einem Fortschreiten des Glaukoms.

### Beispiel 2 (Glaukom)

Es wurden die o. g. Kapseln verwendet.
Die Behandlung wurde an einer Patientin durchgeführt die seit über 15 Jahren an einem fortschreitenden Glaukom erkrankt ist und konservativ behandelt wird.
Die Patientin wurde 5 Jahr lang mit dem genannten Präparat bei Fortführung der konservativen Therapie behandelt. In diesem Zeitraum kam es zu keiner Befundverschlechterung. Die Behandlung schützte somit vor einem Fortschreiten des Glaukoms.

### Beispiel 3 (Fortschreitender Diabetes mellitus mit fortschreitender diabetischer Retinopathie)

Es wurden die o. g. Kapseln verwendet.
Die Behandlung wurde an einem Patienten durchgeführt der seit über 26 Jahren an einem fortschreitenden Diabetes mellitus mit fortschreitender diabetischer Retinopathie erkrankt ist, konservativ behandelt wird und mit wiederholten Laserbehandlungen der Netzhaut versorgt wurde.
Der Patient wurde 4 Jahre lang mit dem genannten Präparat bei Fortführung der konservativen Therapie behandelt. In diesem Zeitraum kam es zu einer weitgehenden Befundstabilisierung. Die Behandlung schütze somit vor einem Fortschreiten des Diabetes mellitus und der diabetischen Retinopathie.

Anschließend wird der Patient mit einem Kombinationspräparat aus Flupirtine und Vitamin C für zwei Jahre bei Fortführung der konservativen Therapie behandelt. In diesem Zeitraum kommt es zu einer deutlichen Befundverbesserung. Die Kombinationstherapie schützt somit nicht nur vor dem Fortschreiten des Diabetes mellitus und der diabetischen Retinopathie sondern kann sie sogar deutlich verbessern.

### Beispiel 4 (Fortschreitender Diabetes mellitus mit fortschreitender diabetischer Retinopathie)

Es wurden die o. g. Kapseln verwendet.
Die Behandlung wurde an einem Patienten durchgeführt der seit über 22 Jahren an einem fortschreitenden Diabetes mellitus mit fortschreitender diabetischer Retinopathie erkrankt ist, konservativ behandelt wird und mit wiederholten Laserbehandlungen der zentralen und peripheren Netzhaut versorgt wurde.
Der Patient wurde 7 Jahre lang mit dem genannten Präparat bei Fortführung der konservativen Therapie behandelt. In diesem Zeitraum kam es zu einer weitgehenden Befundstabilisierung. Die Behandlung schützte somit vor einem Fortschreiten des Diabetes mellitus und der diabetischen Retinopathie.

Anschließend wird der Patient mit einem Kombinationspräparat aus Flupirtine und Vitamin C für zwei Jahre bei Fortführung der konservativen Therapie behandelt. In diesem Zeitraum kommt es zu einer deutlichen Befundverbesserung. Die Kombinationstherapie schützt somit nicht nur vor dem Fortschreiten des Diabetes mellitus und der diabetischen Retinopathie sondern kann sie sogar deutlich verbessern.

### Beispiel 5 (Altersbedingte Maculopathie)

### (fällt nicht unter die Ansprüche)

Es wurden die o. g. Kapseln verwendet.
Die Behandlung wurde an einer Patientin durchgeführt, die seit über 6 Jahren an einer fortschreitenden altersbedingten Maculopathie erkrankt ist und mit einer Laserbehandlung der Macula versorgt wurde.
Die Patientin wurde 6,5 Jahre lang mit dem genannten Präparat behandelt. In diesem Zeitraum kam es zu einer weitgehenden Befundstabilisierung. Die Behandlung schützte somit vor einem Fortschreiten der altersbedingten Maculopathie.

### Beispiel 6 (Altersbedingte Maculopathie)

### (fällt nicht unter die Ansprüche)

Es wurden die o. g. Kapseln verwendet.
Die Behandlung wurde an einer Patientin durchgeführt, die seit über 8 Jahren an einer fortschreitenden altersbedingten Maculopathie erkrankt ist. Die Patientin wurde 4 Jahre lang mit dem genannten Präparat behandelt. In diesem Zeitraum kam es zu einer Befundstabilisierung. Die Behandlung schützte somit vor einem Fortschreiten der altersbedingten Maculopathie.

### Beispiel 7 (Retinitis pigmentosa mit fortschreitender Maculopathie)

Es wurden die o. g. Kapseln verwendet.
Die Behandlung wurde an einer Patientin durchgeführt, die seit über 14 Jahren an einer Retinitis pigmentosa mit fortschreitender Maculopathie erkrankt ist.
Die Patientin wurde 4 Jahre lang mit dem genannten Präparat behandelt. In diesem Zeitraum kam es zu einer weitgehenden Befundstabilisierung. Die Behandlung schütze somit vor einem Fortschreiten der Retinitis pigmentosa und der Retinitis pigmentosa-bedingten Maculopathie.

### Beispiel 8 (Retinitis pigmentosa)

Es wurden die o. g. Kapseln verwendet.
Die Behandlung wurde an einem Patienten durchgeführt, der seit über 19 Jahren an einer Retinitis pigmentosa erkrankt ist. Der Patient wurde 7 Jahre lang mit dem genannten Präparat behandelt. In diesem Zeitraum kam es zu einer weitgehendem Befundstabilisierung. Die Behandlung schütze somit vor einem Fortschreiten der Retinitis pigmentosa.

## Patentansprüche

1. Pharmazeutisches Präparat enthaltend Flupirtine zur Verwendung in der Therapie und/oder Prophylaxe von Diabetes mellitus, diabetischer Retinopathie, diabetischer Maculopathie, Retinitis pigmentosa und/oder menschlichen Glaukomen, wobei das menschliche Glaukom ein Offenwinkelglaukom ist.

2. Pharmazeutisches Präparat nach Anspruch 1, wobei das pharmazeutische Präparat mindestens einen üblichen Träger und/oder mindestens einen üblichen Hilfsstoff aufweist.

3. Pharmazeutisches Präparat nach einem der vorhergehenden Ansprüche, wobei das pharmazeutische Präparat oral appliziert wird.

4. Pharmazeutisches Präparat nach einem der vorhergehenden Ansprüche, wobei das pharmazeutische Präparat als Tablette oder in einer Kapsel vorliegt.

5. Pharmazeutisches Präparat nach einem der vorhergehenden Ansprüche, wobei das pharmazeutische Präparat als Retard-Applikationsform vorliegt.

6. Pharmazeutisches Präparat nach Anspruch 4 oder 5, wobei die Tablette oder die Kapsel einen magensaftresistenten Überzug aufweist.

7. Pharmazeutisches Präparat nach einem der Ansprüche 1 bis 3, wobei das pharmazeutische Präparat als Trinklösung vorliegt.

8. Pharmazeutisches Präparat nach Anspruch 7, wobei das pharmazeutische Präparat als Brausetablette vorliegt.

9. Pharmazeutisches Präparat nach einem der Ansprüche 1 bis 2, wobei das pharmazeutische Präparat als transdermales therapeutisches System, insbesondere als Pflaster, als Tropflösung, insbesondere als Augentropfen, als Salbe, insbesondere als Augensalbe, als Infusionslösung, als Suppositorien, als Gel und/oder als Medikamententräger, insbesondere als ocusert vorliegt.

10. Kombinationspräparat enthaltend Flupirtine und wenigstens einen weiteren Wirkstoff zur Therapie und/oder Prophylaxe von Diabetes mellitus, diabetischer Retinopathie, diabetischer Maculopathie, Retinitis pigmentosa und/oder menschlichen Glaukomen, wobei das menschliche Glaukom ein Offenwinkelglaukom ist.

11. Verwendung von Flupirtine und wenigstens einem weiteren Wirkstoff zur Herstellung eines Kombinationspräparat zur Therapie und/oder Prophylaxe von Diabetes mellitus, diabetischer Retinopathie, diabetischer Maculopathie, Retinitis pigmentosa und/oder menschlichen Glaukomen, wobei das menschliche Glaukom ein Offenwinkelglaukom ist.

12. Verwendung von Flupirtine zur Herstellung eines pharmazeutischen Präparats zur Therapie und/oder Prophylaxe von Diabetes mellitus, diabetischer Retinopathie, diabetischer Maculopathie, Retinitis pigmentosa und/oder menschlichen Glaukomen, wobei das menschliche Glaukom ein Offenwinkelglaukom ist.

13. Flupirtine zur Verwendung in der Therapie und/oder Prophylaxe von Diabetes mellitus, diabetischer Retinopathie, diabetischer Maculopathie, Retinitis pigmentosa und/oder menschlichen Glaukomen, wobei das menschliche Glaukom ein Offenwinkelglaukom ist.

14. Flupirtine und wenigstens ein weiterer Wirkstoff zur Verwendung in der Therapie und/oder Prophylaxe von Diabetes mellitus, diabetischer Retinopathie, diabetischer Maculopathie, Retinitis pigmentosa und/oder menschlichen Glaukomen, wobei das menschliche Glaukom ein Offenwinkelglaukom ist.

## Claims

1. A pharmaceutical preparation containing flupirtine for use in therapy and/or prophylaxis of diabetes mellitus, diabetic retinopathy, diabetic maculopathy, retinitis pigmentosa and/or human glaucomas, wherein the human glaucoma is an open-angle glaucoma.

2. The pharmaceutical preparation according to claim 1,
wherein the pharmaceutical preparation has at least one customary carrier and/or at least one customary excipient.

3. The pharmaceutical preparation according to one of the preceding claims, wherein the pharmaceutical preparation is administered orally.

4. The pharmaceutical preparation according to one of the preceding claims, wherein the pharmaceutical preparation is present in the form of a tablet or in a capsule.

5. The pharmaceutical preparation according to one of the preceding claims, wherein the pharmaceutical preparation is present in the form of a sustained-release form of administration.

6. The pharmaceutical preparation according to claim 4 or 5, wherein the tablet or capsule has a coating that is resistant to gastric juice.

7. The pharmaceutical preparation according to one of claims 1 to 3, wherein the pharmaceutical preparation is present in the form of a drinkable solution.

8. The pharmaceutical preparation according to claim 7,
wherein the pharmaceutical preparation is present in the form of an effervescent tablet.

9. The pharmaceutical preparation according to claim 1 or 2, wherein the pharmaceutical preparation is present in the form of a transdermal therapeutic system, especially a patch, a solution for use im the form of drops, especially eye drops, an ointment, especially an eye ointment, an infusion solution, suppositories, a gel and/or a medicament carrier, especially an ocusert.

10. Combination preparation containing flupirtine and at least one further active ingredient for therapy and/or prophylaxis of diabetes mellitus, diabetic retinopathy, diabetic maculopathy, retinitis pigmentosa and/or human glaucomas, wherein the human glaucoma is an open-angle glaucoma.

11. Use of flupirtine and at least one further active ingredient in manufacture of a combination preparation for therapy and/or prophylaxis of diabetes mellitus, diabetic retinopathy, diabetic maculopathy, retinitis pigmentosa and/or human glaucomas, wherein the human glaucoma is an open-angle glaucoma.

12. Use of flupirtine in manufacture of a pharmaceutical preparation for therapy and/or prophylaxis of diabetes mellitus, diabetic retinopathy, diabetic maculopathy, retinitis pigmentosa and/or human glaucomas, wherein the human glaucoma is an open-angle glaucoma.

13. Flupirtine for use in therapy and/or prophylaxis of diabetes mellitus, diabetic retinopathy, diabetic maculopathy, retinitis pigmentosa and/or human glaucomas, wherein the human glaucoma is an open-angle glaucoma.

14. Flupirtine and at least one further active ingredient for use in therapy and/or prophylaxis of diabetes mellitus, diabetic retinopathy, diabetic maculopathy" retinitis pigmentosa and/or human glaucomas, wherein the human glaucoma is an open-angle glaucoma.

## Revendications

1. Préparation pharmaceutique contenant de la flupirtine, destinée à être utilisée pour la thérapie et/ou la prophylaxie du diabète sucré, de la rétinopathie diabétique, de la maculopathie diabétique, de la rétinite pigmentaire et/ou de glaucomes humains, le glaucome humain étant un glaucome à angle ouvert.

2. Préparation pharmaceutique selon la revendication 1, où la préparation pharmaceutique présente au moins un vecteur usuel et/ou au moins un adjuvant usuel.

3. Préparation pharmaceutique selon l'une des revendications précédentes, où la préparation pharmaceutique est administrée par voie orale.

4. Préparation pharmaceutique selon l'une des revendications précédentes, où la préparation pharmaceutique se présente sous forme de comprimé ou de gélule.

5. Préparation pharmaceutique selon l'une des revendications précédentes, où la préparation pharmaceutique se présente sous une forme à libération prolongée.

6. Préparation pharmaceutique selon la revendication 4 ou la revendication 5, où le comprimé ou la gélule présente un revêtement résistant aux sucs gastriques.

7. Préparation pharmaceutique selon l'une des revendications 1 à 3, où la préparation pharmaceutique se présente sous forme de solution buvable.

8. Préparation pharmaceutique selon la revendication 7,
où la préparation pharmaceutique se présente sous forme de comprimé effervescent.

9. Préparation pharmaceutique selon l'une des revendications 1 ou 2, où la préparation pharmaceutique se présente comme dispositif thérapeutique transdermique, notamment sous forme d'emplâtre, comme solution en gouttes, notamment sous forme de collyre, comme pommade, notamment sous forme de pommade ophtalmique, comme solution pour perfusion, comme suppositoire, comme gel et/ou comme vecteur médicamenteux, notamment sous forme d'insert ophtalmique Ocusert.

10. Association médicamenteuse contenant de la flupirtine et au moins une autre substance active destinée à la thérapie et/ou la prophylaxie du diabète sucré, de la rétinopathie diabétique, de la maculopathie diabétique, de la rétinite pigmentaire et/ou de glaucomes humains, le glaucome humain étant un glaucome à angle ouvert.

11. Utilisation de flupirtine et d'au moins une autre substance active pour la production d'une association médicamenteuse destinée à la thérapie et/ou la prophylaxie du diabète sucré, de la rétinopathie diabétique, de la maculopathie diabétique, de la rétinite pigmentaire et/ou de glaucomes humains, le glaucome humain étant un glaucome à angle ouvert.

12. Utilisation de flupirtine pour la production d'une préparation pharmaceutique destinée à la thérapie et/ou la prophylaxie du diabète sucré, de la rétinopathie diabétique, de la maculopathie diabétique, de la rétinite pigmentaire et/ou de glaucomes humains, le glaucome humain étant un glaucome à angle ouvert.

13. Flurpitine destinée à être utilisée pour la thérapie et/ou la prophylaxie du diabète sucré, de la rétinopathie diabétique, de la maculopathie diabétique, de la rétinite pigmentaire et/ou de glaucomes humains, le glaucome humain étant un glaucome à angle ouvert.

14. Flurpitine, et au moins une autre substance active, destinées à être utilisées pour la thérapie et/ou la prophylaxie du diabète sucré, de la rétinopathie diabétique, de la maculopathie diabétique, de la rétinite pigmentaire et/ou de glaucomes humains, le glaucome humain étant un glaucome à angle ouvert.
